# EUROPÄISCHE PATENTSCHRIFT

(11) **EP 2 714 119 B1**
(45) Veröffentlichungstag und Bekanntmachung des Hinweises auf die Patenterteilung: **16.11.2016**
(21) Anmeldenummer: 12724980.3
(22) Anmeldetag: 30.05.2012
(51) Int. Cl.: A61M 1/00

(54) **VORRICHTUNG ZUR UNTERDRUCKWUNDBEHANDLUNG**
DEVICE FOR VACUUM TREATMENT OF WOUNDS
DISPOSITIF POUR LE TRAITEMENT DE PLAIES PAR DÉPRESSION

(30) Priorität: 01.06.2011 DE 102011110705
(43) Veröffentlichungstag der Anmeldung: 09.04.2014
(73) Patentinhaber: BSN medical GmbH, 20253 Hamburg (DE)
(72) Erfinder: MAY, Alexander, verstorben (DE)
(74) Vertreter: Beetz & Partner mbB
(86) Internationale Anmeldenummer: PCT/EP2012/060101
(87) Internationale Veröffentlichungsnummer: WO 2012/163943

(56) Entgegenhaltungen:
- WO-A2-2009/019414
- DE-A1-102009 038 131
- US-A1- 2002 082 569
- US-A1- 2005 080 384
- US-A1- 2008 294 109
- US-A1- 2009 240 218

## Beschreibung

Die Erfindung betrifft einen Wegwerfkanister sowie eine Unterdruckpumpe für eine Vorrichtung zur Unterdruckwundbehandlung. Das Prinzip einer solchen Behandlung ist beispielsweise in der Patentschrift US 3,572,340 beschrieben. Hierbei entzieht ein durch eine Unterdruckpumpe unter Unterdruck gestellter Behälter einer Wunde, welche durch einen Wundschaum wie zum Beispiel einen offenzelligen Polyurethanschaum ausgefüllt und mittels einer Folie luftdicht versiegelt wurde, über einen Drainageport Wundsekret. Dadurch wird das Wundsekret von der nässenden Wunde abgeleitet und in dem Behälter, der heute üblicherweise als Wegwerfkanister ausgebildet ist, gesammelt. Die Wunde bleibt dabei im Sinne des modernen Wundversorgungsansatzes feucht. Durch die Unterdruckwundtherapie wird die Granulation einer Wunde beschleunigt. Sie schließt sich damit im Schnitt bereits nach ca, 28 Tagen. Sehr großflächige Wunden sind entsprechend länger zu behandeln. Die Unterdruckwundtherapie wird bei tiefen und auch bei flachen (oberflächlichen) Wunden angewandt. Hierzu werden entsprechend verschiedene Wundfüller angeboten. Der von der Pumpe erzeugte Ansaugunterdruck muss der Art der Wunde angepasst werden. Im Normalfall werden Wunden mit einem Unterdruck von 80-120 mmHg (110-160 hPa) behandelt. Unter den möglichen zeitlichen Ansaugdruckmustern kommen vor allem ein kontinuierlicher oder ein intermittierender Sog zum Einsatz. Der kontinuierliche Sog erweist sich im Hinblick auf das Schmerzempfinden der Patienten als vorteilhaft. Das Gewebe wird dabei kontinuierlich behandelt, so dass eine unerwünschte Entspannung des Gewebes ausgeschlossen ist. Der Kanister zur Aufnahme des Wundexsudats wird üblicherweise am Pumpengehäuse so befestigt, dass er an einer Seite des Pumpengehäuses angebracht ist oder zumindest teilweise davon umschlossen wird. Solche Unterdruckwundbehandlungsgeräte sind beispielsweise aus den Patentschriften DE 69505545 T2 für den stationären Einsatz und DE 69629507 T2 für den mobilen Einsatz sowie aus der DE 10 2009 038 131 A1 bekannt. Eine weiterführende Anwendung der Unterdruckwundbehandlung ist in der Patentschrift US 7,931,651 B2 beschrieben.

Die WO 2009/019414 A2 beschreibt einen Wegwerfkanister zur Aufnahme des Wundexsudats bei einer Unterdruckwundtherapie, wobei der Kanister eine Aussparung zur Einführung einer Unterdruckpumpe aufweist, wobei der Kanister und die Unterdruckpumpe so miteinander verbindbar sind, dass die Unterdruckpumpe nur mehr in Einführungsrichtung beweglich ist und wobei ein Verschluss an Kanister und Unterdruckpumpe die Unterdruckpumpe im Kanister gegenüber einer Verschiebung in Einführungsrichtung fixieren kann.

Ein ähnlicher Wegwerfkanister ist ebenfalls aus der US 2009/0240218 A1 bekannt.

Dort werden jeweils Kanister und Unterdruckpumpen über eine profilierte Kontaktfläche miteinander verbunden. Dies geschieht auch bei der DE 102009 038 131, wo Kanister und Pumpe nebeneinander angeordnet sind.

Über die bekannten Bauweisen einer Vorrichtung zur Unterdruckwundbehandlung hinaus besteht Bedarf an einer kostengünstigen Bauweise für ein Wundbehandlungsgerät, das überwiegend stationär, jedoch auch zeitweise mobil einsetzbar ist.

Die Bauart und Position des Kanisters relativ zur Pumpe wirkt sich auf die Wartungsfreundlichkeit beim Auswechseln eines vollen Kanisters, auf die Ablesbarkeit des Füllstands eines zumindest teilweise transparenten Kanisters und auf den Tragekomfort des Unterdruckwundbehandlungsgeräts bestehend aus Pumpe und Kanister am Körper eines Patienten bei seinem mobilen Einsatz aus.

Aufgabe der Erfindung ist es, eine Bauform einer Pumpe und eines Wegwerfkanisters vorzuschlagen, die die genannten Anforderungen im Hinblick auf einen zeitweise mobilen Einsatz vorteilhaft gegenüber dem bekannten Stand der Technik erfüllt.

Die Aufgabe wird gelöst durch einen Wegwerfkanister gemäß Anspruch 1 und eine Unterdruckpumpe gemäß Anspruch 2.

Erfindungsgemäß weist der Wegwerfkanister eine Aussparung auf, in die die Pumpe zumindest teilweise eingesetzt werden kann. Die Wände der Aussparung umschließen die Pumpe soweit, sodass die Pumpe dadurch am Wegwerfkanister in zwei Dimensionen fixiert ist und nur noch in Einführungsrichtung beweglich ist. Durch einen zusätzlichen Verschluss wird die Pumpe bezüglich dieser Bewegungsrichtung am Kanister fixiert. Vorteilhafterweise weist das Pumpengehäuse damit keine schwer der Reinigung zugänglichen Aussparungen mit spitzen Innenwinkeln zur Aufnahme des Kanisters auf. Der grundsätzlich in dieser Bauart schwerer zu reinigende, verwinkelte Kanister wird nach seiner Befüllung statt einer Reinigung entsorgt.

In einer vorteilhaften Ausgestaltung der Erfindung wird der Kanister so geformt und die Aussparung im Kanister so positioniert, dass die Vorrichtung bestehend aus dem Kanister und der darin eingeführten Pumpe, die mit einer Zweipunktaufhängung am Körper des Patienten befestigt werden kann, so ausbalanciert ist, dass die Zugkräfte durch die Gewichtsverteilung der Vorrichtung an beiden Aufhängungspunkten immer ungefähr gleich groß zueinander sind unabhängig von der Befüllung des Kanisters, wenn sich der Kanister in einer normalen waagrechten Betriebslage befindet. Dies erhöht den Tragekomfort am Körper, wenn die Vorrichtung mit einem an beiden Aufhängungspunkten befestigten Gurt getragen wird.

In einer vorteilhaften Ausgestaltung der Erfindung wird dies erreicht, indem der Kanister annähernd symmetrisch zur Spiegelebene ausgeführt ist, die die beiden Aufhängungspunkte ineinander spiegelt, wobei weiter die Aussparung für die Pumpe durch die Spiegelebene ungefähr halbiert wird.

In einer vorteilhaften Ausgestaltung der Erfindung ist der Kanister zumindest an einer Stelle einer Außenwand transparent ausgeführt und mit einer Füllstandsanzeige versehen, so dass sich sein Füllstand dort direkt ablesen lässt.

In einer vorteilhaften Ausgestaltung der Erfindung ist der Kanister zumindest an einer der transparenten Außenwand des Kanisters gegenüberliegenden Stelle der Wand der Aussparung des Kanisters ebenfalls transparent ausgeführt, so dass eine in die Pumpenoberfläche eingelassene Beleuchtung die Füllstandsskala direkt beleuchtet, wenn die Pumpe im Kanister fixiert ist und die Beleuchtung eingeschaltet ist. Dies verbessert die Ablesbarkeit des Füllstands durch eine Erhöhung des Kontrasts bei schlechten Lichtverhältnissen.

In einer vorteilhaften Ausgestaltung der Erfindung wird die Farbe der Beleuchtung in einem sichtbaren Spektralbereich gewählt, der vom Wundexsudat bestmöglich absorbiert wird.

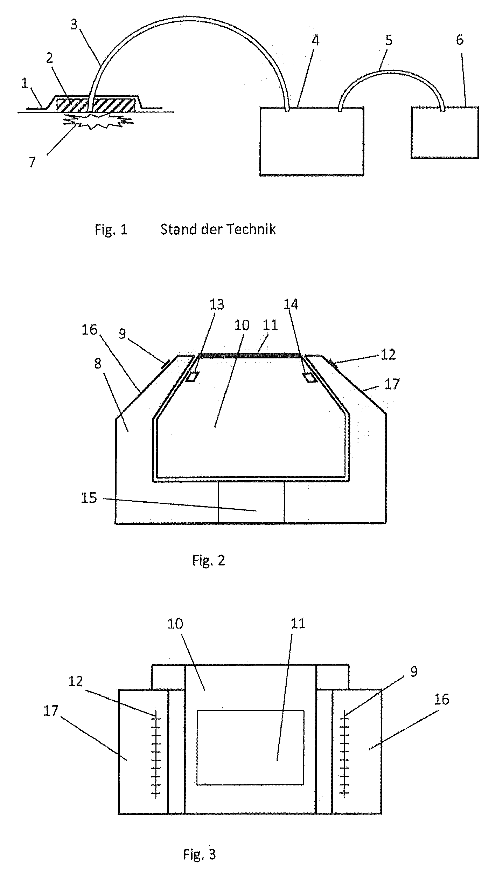

Fig. 1 zeigt das Funktionsprinzip der Unterdruckwundbehandlung. Eine Wunde 7 wird mit einem Wundfüller 2 bedeckt, der mit einer Folie 1 luftdicht abgeschlossen wird, so dass über einen Sog am Drainage-Schlauch 3 das Wundsekret in den Behälter 4 gesaugt wird, der über eine Verbindung 5 durch eine Vakuumpumpe 6 kontrolliert unter Unterdruck gestellt wird.

Fig. 2 zeigt eine bevorzugte beispielhafte Ausführungsform der Erfindung in einer Draufsicht auf das Gerät von oben. Fig. 3 zeigt eine Ansicht der Ausführungsform des Geräts von vorne mit Blick auf das an der Pumpe 10 angebrachte Bedienungsfeld 11 in Form eines Touchpanels. Die Pumpe 10 ist gegen ein Verrutschen in horizontaler Ebene durch einen sie teilweise umschließenden transparenten Kanister 8 gesichert, Der Kanister 8 umschließt hierzu die Pumpe 10 bis auf eine Aussparung zur Bedienung-des Touchpanels 11 von allen Seiten mit Ausnahme der Oberseite, von der die Pumpe in den Kanister eingeführt wird. Durch die an den abgeschrägten Seiten 16, 17 des Kanisters 8 angebrachte Füllstandsskala 9, 12 ist der Füllstand des Wundexsudats sowohl von vorne mit Blick auf das Touchpanel als auch von der Seite her ablesbar. Die zumindest eine Füllstandsskala 9, 12 wird durch eine direkte Beleuchtung 13, 14 an der gegenüberliegenden Seite am Pumpengehäuse bei Bedarf bestrahlt. Die Beleuchtung ist vorzugsweise als LED oder OLED Zelle ausgeführt. In einer beispielhaften bevorzugten Ausführung ist die Dicke des Kanisters 8 an der Stelle der Füllstandsskala 9,12 geringer als die überwiegende sonstige Dicke des Kanisters 8. Dadurch wird eine zu geringe Beleuchtung der Füllstandsskala 9, 12 durch eine zu starke Absorption durch Füllmaterial des Kanisters, das das abgesaugte Wundexsudat bindet, beim Weg des Lichts durch den Kanister 8 vermieden. Um die Pumpe leicht mit einer Hand aus dem Kanister entfernen zu können, weist der Kanister 8 auf der dem Bedienungsfeld 11 der Pumpe abgewandten gegenüberliegenden Seite eine zusätzliche Aussparung 15 auf, durch die die Pumpe mit dem Daumen gefasst werden kann,

in einer weiteren vorteilhaften beispielhaften Ausführung ist die dem Bedienfeld 10 der Pumpe gegenüberliegende Seite des Kanisters, die bei mobilem Betrieb am Körper anliegt, zum Körper der Patienten hin gekrümmt, so dass sich die Auflagefläche am Körper vergrößert, das Gerät besser am Körper fixiert wird und sich der Tragekomfort verbessert.

## Patentansprüche

1. Wegwerfkanister (8) zur Aufnahme des Wundexsudats bei einer Unterdruckwundtherapie, wobei der Kanister (8) eine Aussparung zur Einführung einer Unterdruckpumpe (10) aufweist, wobei der Kanister (8) und die Unterdruckpumpe (10) so miteinander verbindbar sind, dass die Unterdruckpumpe (10) nur mehr in Einführungsrichtung beweglich ist, wobei
der Kanister (8) die Unterdruckpumpe (10) im verbundenen Zustand von allen Seiten, mit Ausnahme der Seite, von der die Unterdruckpumpe in den Kanister eingeführt wird, umschließt, und eine Aussparung zur Bedienung eines Bedienfelds (11), das an der Unterdruckpumpe (10) angebracht ist, aufweist, sowie gegebenenfalls auf der dem Bedienfeld (11) der Unterdruckpumpe (10) abgewandten, gegenüberliegenden Seite eine zusätzliche Aussparung (15) aufweist, durch die die Unterdruckpumpe (10) mit dem Daumen gefasst werden kann, wobei ein Verschluss an Kanister (8) und Unterdruckpumpe (10) die Unterdruckpumpe (10) im Kanister (8) gegenüber einer Verschiebung in Einführungsrichtung fixieren kann, wobei eine an der Unterdruckpumpe (10) angebrachte Beleuchtung (13, 14) so ausgebildet ist, dass sie die an der gegenüberliegenden Außenseite des zumindest dort transparenten Kanisters (8) angebrachte Füllstandsskala (9, 12) direkt beleuchtet.

2. Unterdruckpumpe (10) zum Absaugen eines Wundexsudats bei einer Unterdruckwundtherapie, die ausgebildet ist, in einen Wegwerfkanister (8) zur Aufnahme des Wundexsudats nach Anspruch 1 eingeführt zu werden, wobei die Unterdruckpumpe (10) eine anschaltbare Beleuchtung (13, 14) aufweiset, um die Füllstandsskala (9, 12) des zumindest dort transparenten Wegwerfkanisters (8) zu beleuchten, wenn die Unterdruckpumpe (10) in den Wegwerfkanister (8) eingeführt ist.

## Claims

1. A disposable container (8) for accommodating a wound exudate in a vacuum wound treatment, the container (8) having a recess for insexting a vacuum pump (10), the container(8) and the vacuum pump (10) being connectable to each other in such a way that the vacuum pump (10) is only movable in the insertion direction, wherein
in the c o nne c te d sta te , the c o nta ine r (8) surrounds the vacuum pump (10) from all sides, with the exception of the side from where the vacuum pump in inserted into the container and hasa recess for operating a control panel (11) whichisattached to the vacuum pump (10), as well as optionally has an additional recess (15) on the opposite side facing away from the control panel (11) of the vacuum pump (10), said additional recess serving to grip the vacuum pump (10) with the thumb, wherein a closure arranged at the container(8) and vacuum pump (10) can fix the vac uum pump (10) in the container(8) againstdisplacementin the insertion direction and wherein a light (13, 14) attached to the vacuum pump (10) is made in such a way that it directly illuminates the fill level scale (9, 12) mounted on the opposite outerside of the container(8) which is transparent, a t le a st in this a re a.

2. A vacuum pump (10) for sucking off a wound exudate in a vacuum wound treatment, which is designed to be inverted into a disposable container(8) for accommodating the wound exudate according to claim 1, therein the vacuum pump (10) hasa light(13, 14) thatcan be turned on in order to illuminate the fill level scale (9, 12) of the disposable container (8) which is transparent, at least in this area, when the vacuum pump (10) is inserted into the disposable container (8).

## Revendications

1. Réservoir à usage unique (8) destiné à recueillir l'exsudat de plaies dans le cadre d'un traitement de plaies par dépression, le réservoir (8) présentant un évidement pour l'introduction d'une pompe à vide (10), le réservoir (8) et la pompe à vide (10) pouvant être reliés de telle sorte que la pompe à vide (10) ne soit mobile uniquement dans le sens de son introduction, dans lequel
le réservoir (8) entoure la pompe à vide (10) à l'état relié de tous les côtés, sauf du côté par lequel la pompe à vide est introduite dans le réservoir, et présente un évidement pour l'utilisation d'un panneau de commande (11) placé sur la pompe à vide (10), ainsi que, le cas échéant, un évidement supplémentaire (15) sur le côté opposé au panneau de commande (11) de la pompe à vide (10), à travers lequel il est possible de saisir la pompe à vide (10) avec le pouce, un dispositif de fermeture placé sur le réservoir (8) et la pompe à vide (10) pouvant fixer la pompe à vide (10) dans le réservoir (10) par rapport à une décalage dans le sens de l'introduction, un système éclairage (13, 14) monté sur la pompe à vide (10) étant conçu de façon à éclairer directement la jauge de remplissage (9, 12) se trouvant sur le côté extérieur opposé du réservoir (8), transparent pour le moins à cet endroit-là.

2. Pompe à vide (10) destinée à aspirer l'exsudat de plaies dans le cadre d'un traitement de plaies par dépression, conçue de manière à être introduite dans un réservoir à usage unique (8) destiné à collecter l'exsudat de plaies selon la revendication 1, la pompe à vide (10) présentant un système éclairage activable (13, 14) permettant d'éclairer la jauge de remplissage (9, 12) du réservoir à usage unique (8), transparent pour le moins à cet endroit-là, lorsque la pompe à vide (10) est introduite dans le réservoir à usage unique (8).
